# EUROPEAN PATENT SPECIFICATION

(11) **EP 4 203 832 B1**
(45) Date of publication and mention of the grant of the patent: **24.09.2025**
(21) Application number: 21786597.1
(22) Date of filing: 25.08.2021
(51) Int. Cl.: A61B 34/20, A61B 34/30

(54) **ROBOT CONTROL SYSTEM FOR MULTIPLE ROBOTS**
ROBOTERKONTROLLSYSTEM FÜR MEHRERE ROBOTER
SYSTÈME DE CONTROLLE POUR MULTIPLES ROBOTS

(30) Priority: 26.08.2020 US 202063070591 P
(43) Date of publication of application: 05.07.2023
(73) Proprietor: Mazor Robotics Ltd., 3079567 Caesarea (IL)
(72) Inventor: JUNIO, Dany, 3079567 Caesarea (IL)
(74) Representative: Viering, Jentschura & Partner mbB Patent- und Rechtsanwälte
(86) International application number: PCT/IL2021/051042
(87) International publication number: WO 2022/044009

(56) References cited:
- US-A1- 2020 015 923
- US-A1- 2020 222 122
- BIDAN HUANG ET AL: "A Multi-Robot Cooperation Framework for Sewing Personalized Stent Grafts", ARXIV.ORG, CORNELL UNIVERSITY LIBRARY, 201 OLIN LIBRARY CORNELL UNIVERSITY ITHACA, NY 14853, 8 November 2017 (2017-11-08), XP080835645
- DE ROSSI GIACOMO ET AL: "Cognitive Robotic Architecture for Semi-Autonomous Execution of Manipulation Tasks in a Surgical Environment", 2019 IEEE/RSJ INTERNATIONAL CONFERENCE ON INTELLIGENT ROBOTS AND SYSTEMS (IROS), IEEE, 3 November 2019 (2019-11-03), pages 7827 - 7833, XP033695240, DOI: 10.1109/IROS40897.2019.8967667

## Description

### FIELD

The present technology generally relates to robotic surgery, and relates more particularly to registration of a plurality of robots or robotic arms for robotic surgery.

### BACKGROUND

Surgical navigation systems are used to track the position of one or more objects during surgery. Surgical robots are useful for holding one or more tools or devices during a surgery, and may operate autonomously (e.g., without any human input during operation), semi-autonomously (e.g., with some human input during operation), or non-autonomously (e.g., only as directed by human input). In some situations, use of multiple robotic arms during a surgery can enable more to be accomplished in a shorter period of time than with only one robotic arm.

In addition, US 2020/222122 A1 discloses a system for assisting in guiding and performing a procedure on a subject, wherein the subject may be any appropriate subject such as inanimate object and/or an animate object and wherein the guide and system may include various manipulable or movable members, such as robotic systems, and may be registered to selected coordinate systems.

Further, the publication "Cognitive Robotic Architecture for Semi-Autonomous Execution of Manipulation Tasks in a Surgical Environment" by Giacomo de Rossi et al., published in 2019 IEEE/RSJ international conference on intelligent robots and systems, November 3, 2019, pages 7827-7833, discloses a cognitive robotic architecture that is able to help an operator accomplish a specific task, wherein the architecture integrates an action recognition module to understand the scene, a supervisory control to make decisions, and a model predictive control to plan collision-free trajectory for the robotic arm taking into account obstacles and model uncertainty, and wherein the approach proposed by said publication has been validated on a simplified scenario involving only a da Vinci- surgical robot and a novel manipulator holding standard laparoscopic tools.

Moreover, US 2020/015923 A1 discloses a surgical visualization system, which is configured to identify one or more structure(s) and/or determine one or more distances with respect to obscuring tissue and/or the identified structure(s), wherein the surgical visualization system can facilitate avoidance of the identified structure(s) by a surgical device, wherein the surgical visualization system can comprise a first emitter configured to emit a plurality of tissue-penetrating light waves and a second emitter configured to emit structured light onto the surface of tissue, wherein the surgical visualization system can also include an image sensor configured to detect reflected visible light, tissue-penetrating light, and/or structured light, and wherein the surgical visualization system can convey information to one or more clinicians regarding the position of one or more hidden identified structures and/or provide one or more proximity indicators.
The publication "A Multi-Robot Cooperation Framework for Sewing Personalized Stent Grafts" by Bidan Huang et al. , arXiv.org 2017-11-08, Cornell University Library, 201 Olin Library Cornell University Ithaca, NY 14853, discloses a multi-robot system for manufacturing personalized medical stent grafts. The proposed system adopts a modular design, which includes: a (personalized) mandrel module, a bimanual sewing module, and a vision module. The mandrel module incorporates the personalized geometry of patients, while the bimanual sewing module adopts a learning-by-demonstration approach to transfer human hand-sewing skills to the robots. The human demonstrations were firstly observed by the vision module and then encoded using a statistical model to generate the reference motion trajectories. During autonomous robot sewing, the vision module plays the role of coordinating multirobot collaboration. The system further includes a vision module that is mounted on top of the workspace to record the 6 d.o.f poses of the modules, via bar-code marker mounted on each for visual tracking.

### SUMMARY

In view of the above, the present invention provides a robot control system according to claim 1. Further advantageous embodiments are described in the dependent claims.

The details of one or more aspects of the disclosure are set forth in the accompanying drawings and the description below. Other features, objects, and advantages of the techniques described in this disclosure will be apparent from the description and drawings, and from the claims. Further, the methods disclosed herein do not form part of the invention as such, but are helpful for understanding the claimed invention.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X₁-Xₙ, Y₁-Yₘ, and Z₁-Zₒ, the phrase is intended to refer to a single element selected from X, Y, and Z, a combination of elements selected from the same class (e.g., X₁ and X₂) as well as a combination of elements selected from two or more classes (e.g., Y₁ and Zₒ).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably.

The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

Numerous additional features and advantages of the present invention will become apparent to those skilled in the art upon consideration of the embodiment descriptions provided hereinbelow.

### BRIEF DESCRIPTION OF THE DRAWINGS

The accompanying drawings are incorporated into and form a part of the specification to illustrate several examples of the present disclosure. These drawings, together with the description, explain the principles of the disclosure. The drawings simply illustrate preferred and alternative examples of how the disclosure can be made and used and are not to be construed as limiting the disclosure to only the illustrated and described examples. Further features and advantages will become apparent from the following, more detailed, description of the various aspects, embodiments, and configurations of the disclosure, as illustrated by the drawings referenced below.
Fig. 1 is a block diagram of a system according to at least one embodiment of the present disclosure;
Fig. 2A depicts an operating room with a plurality of robots according to at least one embodiment of the present disclosure;
Fig. 2B depicts an operating room with a plurality of robots according to at least one embodiment of the present disclosure;
Fig. 3 is a flowchart of a method according to an example helpful for understanding the present disclosure;
Fig. 4 is another flowchart of a method according to an example helpful for understanding the present disclosure;
Fig. 5 is another flowchart of a method according to an example helpful for understanding the present disclosure;
Fig. 6 is another flowchart of a method according to an example helpful for understanding the present disclosure; and
Fig. 7 is another flowchart of a method according to an example helpful for understanding the present disclosure.

### DETAILED DESCRIPTION

It should be understood that various aspects disclosed herein may be combined in different combinations than the combinations specifically presented in the description and accompanying drawings. It should also be understood that, depending on the example or embodiment, certain acts or events of any of the processes or methods described herein may be performed in a different sequence, and/or may be added, merged, or left out altogether (e.g., all described acts or events may not be necessary to carry out the disclosed techniques according to different embodiments of the present disclosure). In addition, while certain aspects of this disclosure are described as being performed by a single module or unit for purposes of clarity, it should be understood that the techniques of this disclosure may be performed by a combination of units or modules associated with, for example, a computing device and/or a medical device.

In one or more examples, the described methods, processes, and techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors (e.g., Intel Core i3, i5, i7, or i9 processors; Intel Celeron processors; Intel Xeon processors; Intel Pentium processors; AMD Ryzen processors; AMD Athlon processors; AMD Phenom processors; Apple A10 or 10X Fusion processors; Apple A11, A12, A12X, A12Z, or A13 Bionic processors; or any other general purpose microprocessors), application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

Before any embodiments of the disclosure are explained in detail, it is to be understood that the disclosure is not limited in its application to the details of construction and the arrangement of components set forth in the following description or illustrated in the drawings. The disclosure is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items. Further, the present disclosure may use examples to illustrate one or more aspects thereof. Unless explicitly stated otherwise, the use or listing of one or more examples (which may be denoted by "for example," "by way of example," "e.g.," "such as," or similar language) is not intended to and does not limit the scope of the present disclosure.

Use of a plurality of robotic arms during a robotic surgery may enable increasingly complex autonomous robotic surgeries, as well as increasingly complex robot-assisted surgeries. Some surgeries and some surgical tasks require multiple simultaneous contacts with a patient, and such surgeries and surgical tasks typically cannot be completed autonomously using a single robotic arm. Moreover, while routine tasks tend to be preferred candidates for automation, routine tasks that requires multiple simultaneous contacts often cannot readily be automated without the simultaneous use of multiple robotic arms.

Two issues that arise in connection with the simultaneous use of multiple robotic arms are the feasibility and complexity of tracking multiple robotic arms with different references frames and/or with the use of patient mounted reference frames. Where each arm requires a separate reference frame to correlate the robotic coordinate system with the patient anatomy (sometimes referred to as the patient space or patient coordinate system), the surgical field becomes increasingly crowded, and the time required for robot set-up increases due to the need to register each robot to its respective reference frame. Where such reference frames are patient-mounted, additional reference frames require additional incisions, thus causing increased trauma to the patient and increasing the risk of future infection and/or complications.

**In** some embodiments of the present disclosure, to reduce surgical field crowding and otherwise address these and other issues, one robotic arm may be used as a reference frame (e.g., in connection with navigation technology) for one or more additional robotic arms. The creation of the reference frame could be a detection of navigation markers of any type on one or more of the arms individually (e.g., navigation markers on one or more segments of one robotic arm), or could be done by creating a larger scale, real-time changing reference frame between several arms in the same system (e.g., navigation markers on one or more segments of multiple robotic arms).

As discussed herein, some robotic systems may comprise a single, common hub supporting a plurality of robotic arms. The use of such systems may not necessarily require multiple reference frames (e.g., one reference frame per arm), because once one of the plurality of robotic arms has been registered to a reference frame (so as to correlate a coordinate system of the robotic system with the patient space), the common hub may be configured to control each of the plurality of robotic arms based on that registration. For example, if the common hub is able to track or otherwise determine a position of each of the plurality of robotic arms within the robotic system coordinate system, then only a single registration of the robotic system coordinate system to the patient coordinate system may be needed. In other words, a single common hub with multiple robotic arms may use a single robotic coordinate system in which the robotic system controls and directs movements of both arms.

In other instances, a plurality of separate robotic systems (each having, for example, a separate base and one or more robotic arms) may be used. For example, these robotic systems may each be independently movable relative to the other robotic systems. In these examples, each robotic system may utilize an independent coordinate system, such that registration of a coordinate system of a first robotic system to a patient coordinate system may not be effective alone for correlating a coordinate system of a second robotic system to the patient coordinate system.

According to some embodiments of the present disclosure, a first robotic coordinate system may be correlated to a navigation coordinate system and/or to a patient coordinate system, and a second robotic space may be correlated to the first robotic coordinate system. The second robotic coordinate system may be correlated to the first robotic coordinate system based on a physical connection between the robots corresponding to the first and second robotic coordinate systems, or the correlation may be based on a sensed or otherwise detected relationship between the first and second robotic coordinate systems.

In still other embodiments, a first robotic coordinate system may be correlated to a navigation coordinate system and/or to a patient coordinate system using a first reference frame, and a second robotic coordinate system may also be correlated to a navigation coordinate system and/or to a patient coordinate system use the first reference frame.

Embodiments of the present disclosure provide technical solutions to the problems of (1) registering a plurality of robots and/or robotic arms to a navigation coordinate system and/or a patient coordinate system; (2) using only a single reference frame for such registration so as to avoid crowding the surgical field with a plurality of reference frames; (3) safely operating a plurality of robots in a surgical environment; (4) avoiding a collision or other interference between multiple robots working in a common surgical space by registering a coordinate system of each robot to a common patient and/or navigation space; and/or (5) completing setup of a robotic system for use in a surgical procedure as efficiently as possible.

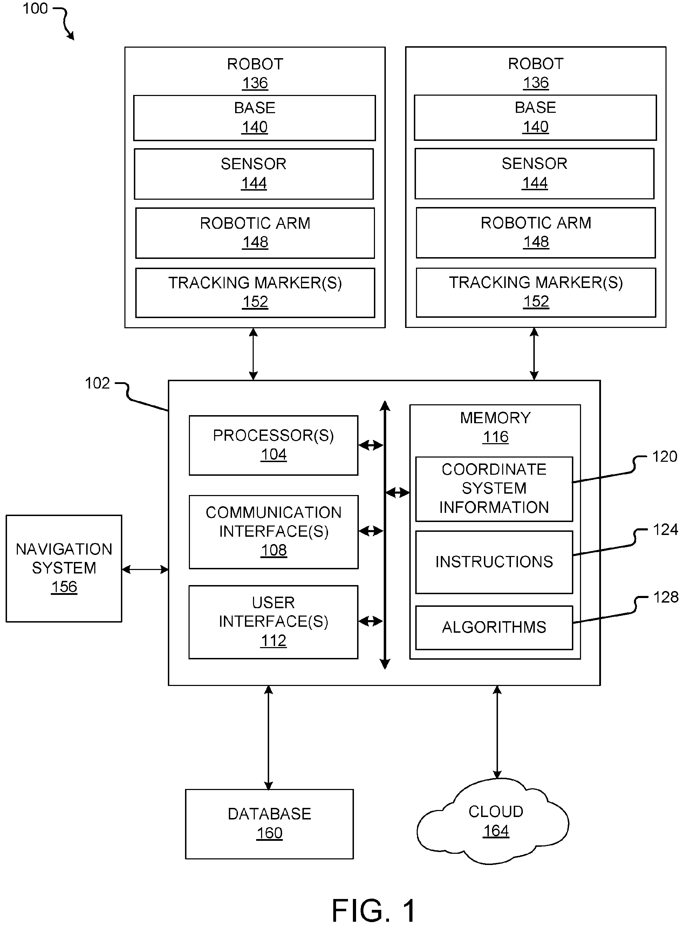

Turning first to Fig. 1, a block diagram of a system 100 according to at least one embodiment of the present disclosure is shown. The system 100 may be used, for example: to carry out one or more aspects of one or more of the methods disclosed herein; for navigation purposes; for registration purposes; to carry out a fully autonomous and/or a robot-assisted surgery using a plurality of robots; or for any other useful purpose. The system 100 comprises a computing device 102, at least two robots 136, a navigation system 156, a database 160, and a cloud 164. Notwithstanding the foregoing, systems according to other embodiments of the present disclosure may omit any one or more of the computing device 102, one or more of the at least two robots 136, the navigation system 156, the database 160, and/or the cloud 164. Additionally, systems according to other embodiments of the present disclosure may arrange one or more components of the system 100 differently (e.g., one or more of the robots 136 and/or the navigation system 156 may comprise one or more of the components shown in Fig. 1 as being part of the computing device 102).

The computing device 102 comprises at least one processor 104, at least one communication interface 108, at least one user interface 112, and at least one memory 116. A computing device according to other embodiments of the present disclosure may omit one or both of the communication interface(s) 108 and the user interface(s) 112.

The at least one processor 104 of the computing device 102 may be any processor identified or described herein or any similar processor. The at least one processor 104 may be configured to execute instructions stored in the at least one memory 116, which instructions may cause the at least one processor 104 to carry out one or more computing steps utilizing or based on data received, for example, from the robot 136, the navigation system 156, the database 160, and/or the cloud 164.

The computing device 102 may also comprise at least one communication interface 108. The at least one communication interface 108 may be used for receiving image data or other information from an external source (such as a robot 136, the navigation system 156, the database 160, the cloud 164, and/or a portable storage medium (e.g., a USB drive, a DVD, a CD)), and/or for transmitting instructions, images, or other information from the at least one processor 104 and/or the computing device 102 more generally to an external system or device (e.g., another computing device 102, a robot 136, the navigation system 156, the database 160, the cloud 164, and/or a portable storage medium (e.g., a USB drive, a DVD, a CD)). The at least one communication interface 108 may comprise one or more wired interfaces (e.g., a USB port, an ethernet port, a Firewire port) and/or one or more wireless interfaces (configured, for example, to transmit information via one or more wireless communication protocols such as 802.11a/b/g/n, Bluetooth, Bluetooth low energy, NFC, ZigBee, and so forth). In some embodiments, the at least one communication interface 108 may be useful for enabling the device 102 to communicate with one or more other processors 104 or computing devices 102, whether to reduce the time needed to accomplish a computing-intensive task or for any other reason.

The at least one user interface 112 may be or comprise a keyboard, mouse, trackball, monitor, television, touchscreen, button, joystick, switch, lever, and/or any other device for receiving information from a user and/or for providing information to a user of the computing device 102. The at least one user interface 112 may be used, for example, to receive a user selection or other user input in connection with any step of any method described herein; to receive a user selection or other user input regarding one or more configurable settings of the computing device 102, one or more of the robots 136, and/or of another component of the system 100; to receive a user selection or other user input regarding how and/or where to store and/or transfer data received, modified, and/or generated by the computing device 102; and/or to display information (e.g., text, images) and/or play a sound to a user based on data received, modified, and/or generated by the computing device 102. Notwithstanding the inclusion of the at least one user interface 112 in the system 100, the system 100 may automatically (e.g., without any input via the at least one user interface 112 or otherwise) carry out one or more, or all, of the steps of any method described herein.

Although the at least one user interface 112 is shown as part of the computing device 102, in some embodiments, the computing device 102 may utilize a user interface 112 that is housed separately from one or more remaining components of the computing device 102. In some embodiments, the user interface 112 may be located proximate one or more other components of the computing device 102, while in other embodiments, the user interface 112 may be located remotely from one or more other components of the computer device 102.

The at least one memory 116 may be or comprise RAM, DRAM, SDRAM, other solid-state memory, any memory described herein, or any other tangible non-transitory memory for storing computer-readable data and/or instructions. The at least one memory 116 may store information or data useful for completing, for example, any step of the methods 300, 400, 500, 600, and/or 700 described herein. The at least one memory 116 may store, for example, information about one or more coordinate systems 120 (e.g., information about a robotic coordinate system or space corresponding each robot 136, information about a navigation coordinate system or space, information about a patient coordinate system or space); instructions 124 for execution by the at least one processor 104, for example to cause the at least one processor 104 to carry out one or more of the steps of the methods 300, 400, 500, 600, and/or 700; and/or one or more algorithms 128 for use by the processor in carrying out any calculations necessary to complete one or more of the steps of the methods 300, 400, 500, 600, and/or 700 (e.g., to map one coordinate system to another coordinate system), or for any other calculations. Such predetermined coordinate system(s) information 120, instructions 124, and/or algorithms 128 may, in some embodiments, be organized into one or more applications, modules, packages, layers, or engines, and may cause the at least one processor 104 to manipulate data stored in the at least one memory 116 and/or received from or via another component of the system 100.

Each of the robots 136 may be any surgical robot or surgical robotic system. Each robot 136 may be or comprise, for example, the Mazor X^{™} Stealth Edition robotic guidance system. Each robot 136 may comprise a base 140 that supports a robotic arm 148. Each robot 136 may comprise one or more robotic arms 148 (e.g., some robots 136 may comprise two robotic arms 148, three robotic arms 148, four robotic arms 148, or another number of robotic arms 148). Each robotic arm 148 may, in some embodiments, assist with a surgical procedure (e.g., by holding a tool in a desired trajectory or pose and/or supporting the weight of a tool while another robot 136, a surgeon, or another medical professional operates the tool; or by holding a patient's skin taut while another robot 136, a surgeon, or other medical professional cuts an incision in the patient's skin; or otherwise) and/or automatically carry out a surgical procedure.

Each robotic arm 148 may have three, four, five or six degrees of freedom.

The robot 136 also comprises one or more sensors 144. The sensor 144 may be an imaging sensor, such as a visible light camera, an infrared camera, or an ultrasound probe.

Data from the sensor(s) 144 may be provided to a processor of the robot 136, to the processor 104 of the computing device 102, and/or to the navigation system 156. The data may be used to calculate a position in space of the robotic arm 148 relative to one or more coordinate systems (e.g., based on coordinate system information 120 stored in the memory 116). The calculation may be based not just on data received from the sensor(s) 144, but also on data or information (such as, for example, physical dimensions) about, for example, a robot 136 or a portion thereof, or any other relevant object, which data or information may be stored, for example, in a memory 116 of a computing device 102 or in any other memory.

One or more tracking markers 152 are fixedly secured to or positioned on the robot 136, whether on the base 140, the robotic arm 148, and/or elsewhere. As used herein, "fixedly secured" does not mean "permanently secured," and indeed the tracking markers 152 may be detachable from the robot 136. The tracking markers 152 may be light-emitting diodes (LEDs). The tracking markers 152 may all be identical, or one or more of the tracking markers 152 may be different than another one or more of the tracking markers 152. In some embodiments, one or more of the tracking markers 152 may be configured to emit light at a first wavelength, and another one or more of the tracking markers 152 may be configured to emit light at a second wavelength different than the first wavelength. Also in some embodiments, one or more of the tracking markers 152 may be configured to reflect light at a first wavelength, while another one or more of the tracking markers 152 may be configured to reflect light at a second wavelength that is different than the first wavelength. The emitted and/or reflected wavelengths of light of the embodiments described above may be wavelengths within a particular spectrum (e.g., wavelengths corresponding to red light versus wavelengths corresponding to blue light in the visible spectrum, or different wavelengths in the infrared spectrum) as well as wavelengths from different spectrums (e.g., a wavelength in the visible spectrum versus a wavelength in the infrared spectrum).

In some embodiments, one or more of the tracking markers 152 may be or comprise an LED that pulses at a first frequency, and another one or more of the tracking markers 152 may be or comprise an LED that pulses at a second frequency different than the first frequency. In some embodiments, the tracking markers 152 may be or comprise reflective spheres, geometric patterns (such as, for example, QR codes), or other items or features that may be readily distinguished by a sensor such as the sensor 144 and/or a navigation system such as the navigation system 156.

In some embodiments of the present disclosure, one or more of a plurality of tracking markers 152 may be moveably secured to the robotic arm 148, and may further be selectively moveable relative to the robotic arm 148. In such embodiments, one or more of the plurality of tracking markers 152 may be configured to move (or to be moved automatically) from a first position on the robotic arm 148 to a second position on the robotic arm 148 when the robotic arm 148 moves into or out of a certain position or set of positions. The purpose of such movement of the one or more of the plurality of tracking markers 152 may be to facilitate maintenance of a line of sight between each (or at least a subset) of the plurality of tracking markers 152 and a sensor 144 or a navigation system 156. In such embodiments, the robot 136 (and/or another component of the system 100) may be configured to track whether each of the plurality of tracking markers 152 is in its respective first position or second position, and to provide such information to the navigation system 156 (or to any other component of the system 100) to enable correlation of a robotic coordinate system with a navigation coordinate system (or any other coordinate system) based on a position of the tracking markers 152 relative to the robotic arm 148 as known by the robot 136 (and/or another component of the system 100), and further based on a position of the tracking markers 152 as detected by the sensor 144 and/or the navigation system 156.

Use of the tracking markers 152 to determine a position in space of a robotic arm 148 is described more fully in U.S. Patent Application No. 63/036,130, filed on June 8, 2020, and entitled "Robotic Reference Frames for Navigation,".

The navigation system 156 of the system 100 may provide navigation for a surgeon and/or for the two or more robots 136 during an operation. The navigation system 156 may be any now-known or future-developed navigation system, including, for example, the Medtronic StealthStation^{™} S8 surgical navigation system. The navigation system 156 may include a camera or other sensor(s) for detecting and/or tracking one or more reference markers, navigated trackers, or other objects within an operating room or other room where a surgical procedure takes place. In various embodiments, the navigation system 156 may be used to track a position of the robotic arm 148 (or, more particularly, of one or more tracking markers 152 attached to the robotic arm 148) of each robot 136. The navigation system 156 may be used to track a position of one or more reference frames, markers, or arrays or other structures useful for detection by a camera or other sensor of the navigation system 156. The navigation system 156 may be used, for example, to detect a position of a reference frame mounted to a patient and/or a position of one or more robotic arms 148, and to register or otherwise correlate a patient coordinate system to a robotic coordinate system based at least on the detected positions. The navigation system 156 may include a display for displaying one or more images from an external source (e.g., the computing device 102, a database 160, the cloud 164, or another source) or a video stream from the camera or other sensor of the navigation system 156. In some embodiments, the system 100 may operate without the use of the navigation system 156.

The database 160 may store information that correlates one coordinate system to another (e.g., one or more robotic coordinate systems to a patient coordinate system and/or to a navigation coordinate system). The database 160 may additionally or alternatively store, for example, information about or corresponding to one or more characteristics of the tracking markers 152; one or more surgical plans (including, for example, image information about a patient's anatomy at and/or proximate the surgical site, for use by the robots 136, the navigation system 156, and/or a user of the computing device 102 or of the system 100); one or more images useful in connection with a surgery to be completed by or with the assistance of one or more other components of the system 100; and/or any other useful information. The database 160 may be configured to provide any such information to the computing device 102 or to any other device of the system 100 or external to the system 100, whether directly or via the cloud 164. In some embodiments, the database 160 may be or comprise part of a hospital image storage system, such as a picture archiving and communication system (PACS), a health information system (HIS), and/or another system for collecting, storing, managing, and/or transmitting electronic medical records including image data.

The cloud 164 may be or represent the Internet or any other wide area network. The computing device 102 may be connected to the cloud 164 via the communication interface 108, using a wired connection, a wireless connection, or both. In some embodiments, the computing device 102 may communicate with the database 160 and/or an external device (e.g., a computing device) via the cloud 164.

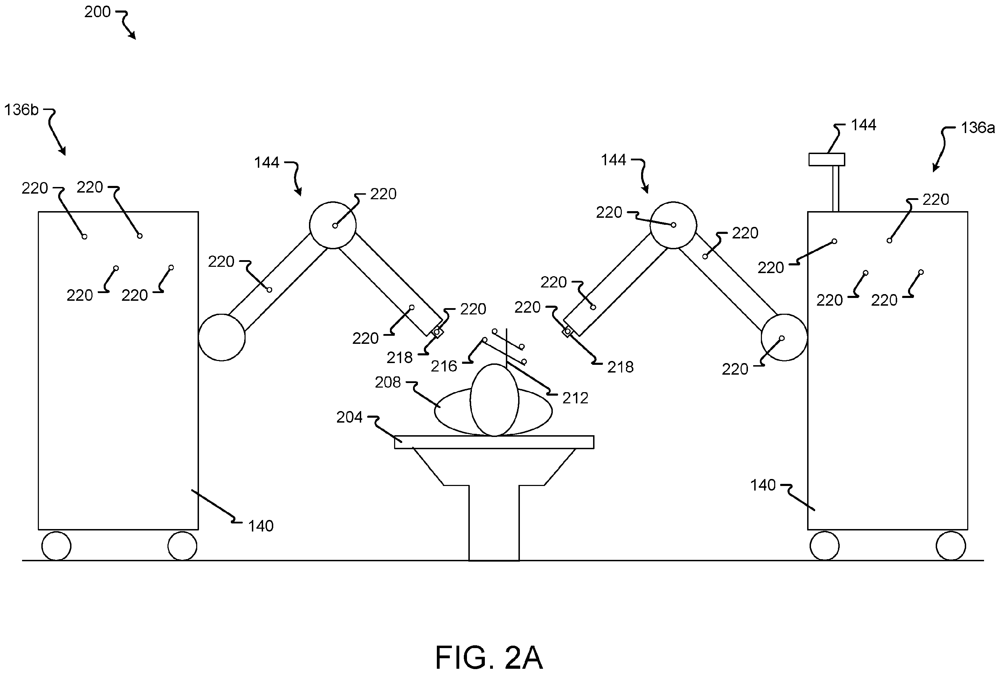

Turning now to Fig. 2A, embodiments of the present disclosure may be used, for example, in connection with a robot-assisted or completely autonomous surgery involving two robots 136a, 136b. Although the two robots 136a, 136b may be positioned anywhere in an operating room 200, Fig. 2A shows the two robots 136a, 136b positioned on opposite sides of an operating table 204, on which a patient 208 rests. A reference frame 212 is fixedly secured to the patient 208, and more specifically to an anatomical element of the patient such as a bone (e.g., a pelvis, a vertebra, a skull). The reference frame 212 comprises a plurality of reflective spheres 216, although in some embodiments the reference frame 212 may comprise one or more other high-visibility markers that may be easily detected by a navigation system camera or other sensor (such as infrared-emitting diodes). The reference frame 212 is detectable by a navigation system camera, electromagnetic sensor, or other suitable sensor for navigation systems other than optical and electromagnetic navigations systems, respectively. The reference frame 212 therefore enables a navigation system (e.g., the navigation system 156) to determine a precise pose (e.g., a precise position and orientation) of the reference frame 212 in a coordinate system of the navigation system. Using detected information about the pose of the reference frame 212 as well as image information about the anatomical element to which the reference frame is attached and/or the precise position of attachment of the reference frame to the anatomical element, a computing device such as the computing device 102 and/or the navigation system 156 is able to register or otherwise correlate a patient-centric coordinate system to the coordinate system of the navigation system, or vice versa.

As shown in Fig. 2A, each of the robots 136a, 136b comprises a mobile base 140 and a robotic arm 148, and each robotic arm 148 comprises an end effector 218. The mobile base 140 is selectively movable, and once positioned in a desired location can be locked into place (whether using wheel locks or otherwise) to prevent movement thereof during surgery. At any point during a surgery (if necessary), and/or at the conclusion of the surgery, the mobile base 140 can be unlocked to facilitate removal and/or repositioning of the robots 136a, 136b. Although shown as stand-alone, wheeled robots in Fig. 2A, the robots 136a, 136b may alternatively be mounted (directly or indirectly) to the operating table, or to another secure (e.g., immobile) structure, including for example an operating room floor, ceiling, wall, and/or any structure securely mounted to any of the foregoing.

A plurality of tracking markers 220 are positioned on the robots 136, including some on the bases 140 and others on the robotic arms 148 (including one tracking marker 220 on each end effector 218). The tracking markers 220 may be the same as or similar to the tracking markers 152. The positions of the tracking markers 220 on the robots 136a, 136b in Fig. 2A are example positions only; in different embodiments one or more tracking markers 220 may be located in a different position on either or both of the robots 136a, 136b. Additionally, in different embodiments more or fewer tracking markers 220 may be provided on the robots 136a, 136b. For example, in some embodiments, each robot 136a, 136b may comprise only a single tracking marker 220, which may be, for example, on an end effector 218 of the robotic arm 148. In other embodiments, each robot 136a, 136b may comprise tracking markers 220 positioned only on the robotic arm 148, or only on the base 140. Also in some embodiments, tracking markers 220 may be positioned only on the joints of the robotic arms 148, or only on the segments of the robotic arms 148 that extend between the joints thereof (or between the end effector 218 and a joint of the robotic arm 148). The tracking markers 220 may allow a computing device (e.g., the computing device 102) and/or a navigation system (e.g., the navigation system 156) to locate the robots 136 (or the robotic arms 148, or the end effector 218) in navigation space (e.g., in a navigation coordinate system), and/or in patient space (e.g., in a patient coordinate system).

Moreover, in some embodiments, the tracking markers 220 may enable a computing device and/or navigation system to register or otherwise correlate a coordinate system corresponding to a robotic space of each robot 136a, 136b to one or both of a patient coordinate system (corresponding to a patient space) and/or a navigation coordinate system (corresponding to a navigation space). The registration or other correlation may occur simultaneously for both robots 136a, 136b, or in sequence. In some embodiments, the coordinate systems of each robot 136a, 136b as well as the patient coordinate system of a patient space corresponding to the patient 208 may be registered or correlated to each other, and/or to a navigation coordinate system, simultaneously.

In some embodiments, registration or other correlation of the coordinate systems of the robots 136a, 136b and the patient space of the patient 208 may enable, for example, a robotic control system (which may be, for example, a computing device such as the computing device 102, or a navigation system such as the navigation system 156, or may comprise both a computing device and a navigation system) to control both robots 136a, 136b simultaneously so as to maintain one robot 136a (including the robotic arm 148 thereof) in a first work volume, and the other robot 136b (including the robotic arm 148 thereof) in a second work volume separate from (but possibly adjacent to) the first work volume. Where the first and second work volumes are adjacent to each other, for example, and/or have a more complex shared boundary than a simple plane, registration of the coordinate systems as discussed above may beneficially facilitate accurate definition of the first and second work volumes, and thus may help to ensure successful separation of the robotic arms 148 of the robots 136a, 136b, and prevention of any interference therebetween.

As one example, a robotic control system as described above may control a pair of robots 136a, 136b to operate in separate, non-adjacent work volumes. One robot 136a could be maintained within a work volume encompassing an upper vertebra (e.g., a vertebra of the thoracic spine) and the other robot 136b could be maintained within a work volume encompassing a lower vertebra (e.g., a vertebra of the lumbar spine), where the upper and lower vertebra are not adjacent to each other. As another example, the robotic control system may control the pair of robots 136a, 136b to operate in separate but adjacent work volumes. Thus, one robot 136a could be maintained within a work volume encompassing a first vertebra, and the other robot 136b could be maintained with a work volume encompassing a second vertebra that is adjacent to the first vertebra. Also in some embodiments, the boundary between the work volume of each robot 136 may not be defined by an anatomical boundary. For example, one robot 136a may be controlled within a work volume that encompasses one half of a vertebra, and the other robot 136b may be controlled within a work volume that encompasses the other half of the same vertebra.

**In** other embodiments, registration or other correlation of the coordinate systems of the robots 136a, 136b and the patient space of the patient 208 may enable a robotic control system (which may be, for example, a computing device such as the computing device 102, or a navigation system such as the navigation system 156, or may comprise both a computing device and a navigation system) to control both robots 136a, 136b simultaneously within a common work volume to accomplish one or more individual surgical tasks and/or to complete a surgical procedure without interference with each other, despite the robotic arms 148 and/or the end effectors 218 alternately occupying the same space within the common work volume. In other words, rather than limit one robot 136a, 136b to operating on one side of the patient 208 and the second robot 136a, 136b to operating on the other side of the patient 208, registration of the coordinate systems of the robots 136a, 136b to a coordinate system corresponding to the patient 208 may enable more efficient use of the robots 136 (e.g., without artificially limiting potential movement paths of the robotic arms 148) than would be possible without registration (although this is not always the case, and as discussed above, embodiments of the present disclosure may limit each robot 136a, 136b to separate work volumes). Registration also ensures that each robot 136a, 136b can interact with precisely the intended portion of the patient 208, thus preserving patient safety and ensuring that any surgical task or procedure is accomplished properly. Where the registration comprises a registration or other correlation to a navigation space, the registration further enables the navigation system to track a precise position of each robot 136a, 136b relative to the patient 208, and/or to provide information and/or commands useful for controlling the robots 136a, 136b to accomplish a given surgical task or procedure without interfering with each other and/or without harming the patient 208.

The robot 136a comprises a sensor 144, which may be used in some embodiments to detect a position of the robot 136b and/or of the robotic arm 148 of the robot 136b (e.g., by detecting a position of the tracking markers 220 on the robot 136 and/or the robotic arm 148 of the robot 136b). The sensor 144 may be used, for example, to facilitate registration or other correlation of the robotic coordinate system of the robot 136b to the robotic coordinate system of the robot 136a or vice versa. Thus, once a first coordinate system of one of the robots 136a, 136b has been registered to a patient coordinate system corresponding to the patient 208, and/or to a navigation coordinate system corresponding a navigation system such as the navigation system 156, a second coordinate system of the other of the robots 136a, 136b may be registered or otherwise correlated to the first coordinate system based on information gathered by the sensor 144.

Although the present disclosure encompasses embodiments in which a first registration is completed between a first coordinate system of a first robot 136a and then a second registration is completed between the first coordinate system of the first robot 136a and a second coordinate system of the second robot 136a, as discussed above, in other embodiments of the present disclosure each robot 136a, 136b is registered directly to the patient coordinate system corresponding to the patient 208, and/or to a navigation coordinate system corresponding to a navigation system such as the navigation system 156. In such embodiments, a sensor 144 on the robot 136a may not be needed for registration purposes, or at least for purposes of registering a first coordinate system of the first robot 136a to a second coordinate system of the second robot 136b.

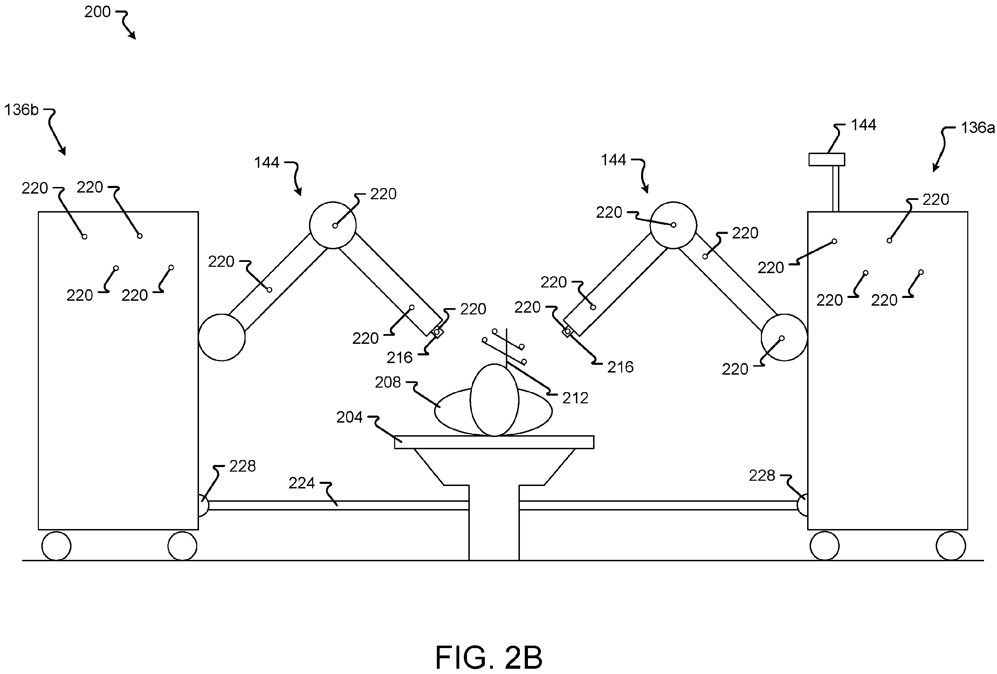

Fig. 2B is largely identical to Fig. 2A, except that in Fig. 2B, the two robots 136 are physically connected to each other with a rigid, elongate member 224 that is secured to each robot 136a, 136b at a joint 228. The elongate member 224 may be, for example, a metal bar, or a bar or other member made of any rigid material. The elongate member 224 may comprise a ball at each end thereof, and the joint 228 on each robot 136a, 136b may comprise a socket adapted to receive the ball, as well as a locking mechanism configured to lock the ball into the socket at a given orientation, so as to prevent relative movement of the member 224, and thus of the robots 136a, 136b, once the member 224 is locked in position. By so connecting the robots 136a, 136b, a coordinate system of one robot 136a, 136b can be registered or correlated to a coordinate system of the other robot 136a, 136b based on, for example, dimensional information about the member 224 (e.g., a length of the member 224) and sensed or otherwise obtained information about an angle at which the member 224 is secured to the joint 228/robot 136. Such information may be sensed, for example, by a sensor in the joint 228, or may be measured using markings on the joint 228 or using a separate gauge or tool. In some embodiments, measurements along six degrees of freedom may be needed to accurately register or correlate the coordinate systems of two connected robots 136a, 136b, but in other embodiments (depending, e.g., on whether the robots 136a, 136b are identical, a location of the joint 228 on each robot 136a, 136b, etc.) measurements along fewer than six degrees of freedom may be necessary. For example, in some embodiments, measurements along five, four, three, or two degrees of freedom may be sufficient.

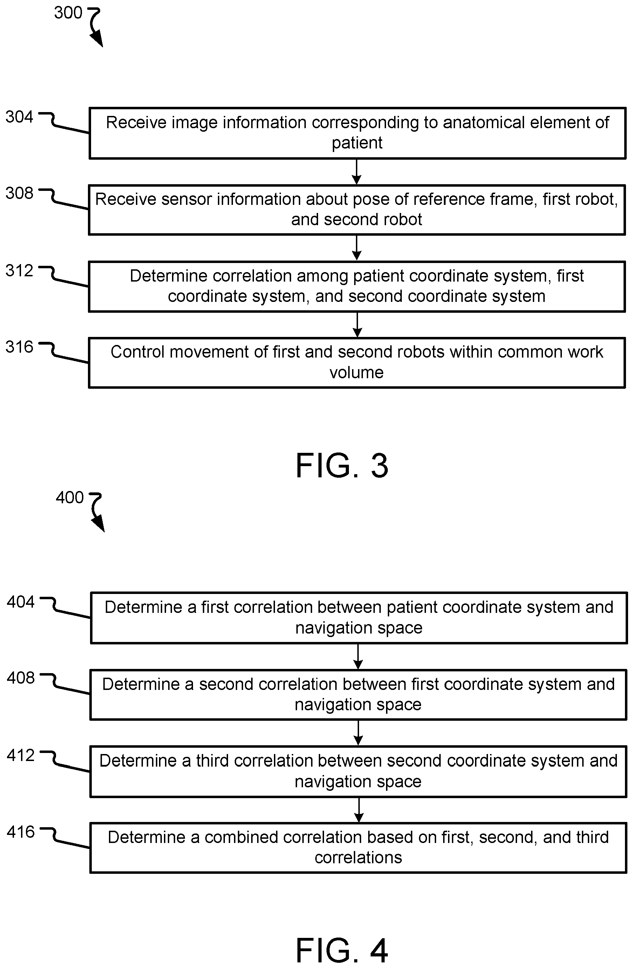

Fig. 3 depicts a registration method 300. The registration method 300 (and/or one or more steps thereof) may be carried out or otherwise performed, for example, by at least one processor. The at least one processor may be the same as or similar to the processor(s) 104 of the computing device 102 described above. The at least one processor may be part of a robot (such as a robot 136) or part of a navigation system (such as a navigation system 156). A processor other than any processor described herein may also be used to execute the method 300. The at least one processor may perform the method 300 by executing instructions stored in a memory, such as the instructions 124 of the memory 116. The instructions may correspond to one or more steps of the method 300 described below. The instructions may cause the processor to execute one or more algorithms, such as the algorithms 128. For example, one or more such algorithms 128 may be used to map one coordinate system to another coordinate system once each coordinate system has been located.

The method 300 comprises receiving image information corresponding to an anatomical element of a patient (step 304). The image information may be received, for example, as part of a preoperative plan. Additionally or alternatively, the image information may be received, whether directly or indirectly, from an imaging device such as a CT scanner, a magnetic resonance imaging (MRI) scanner, an optical coherence tomography (OCT) scanner, an O-arm (including, for example, an O-arm 2D long film scanner), a C-arm, a G-arm, another device utilizing X-ray-based imaging (e.g., a fluoroscope or other X-ray machine), or any other imaging device. The image information may be or comprise a plurality of two-dimensional (2D) images, and/or one or more three-dimensional (3D) images. In some embodiments, the image information may be or comprise a 3D model of the anatomical element, which may in turn have been generated using one or more 2D or 3D images.

The method 300 also comprises receiving sensor information about a pose of a reference frame, a first robot, and a second robot (step 308). The reference frame may be the same as or similar to the reference frame 212, and the first and second robots may be the same as or similar to, for example, the robots 136 of Fig. 1, and/or the robots 136a, 136b of Figs. 2A-2B. In some embodiments, the pose of the reference frame, first robot, and second robot may be a simultaneous pose (e.g., a pose of the reference frame, first robot, and second robot at the same instant in time). In other embodiments, the pose of the reference frame, first robot, and second robot may be a pose of each individual device at different moments in time. The sensor information may be any information useful for determining the pose of the reference frame, the first robot, and the second robot at a single point in time. Thus, for example, the sensor information may comprise image information received, whether directly or indirectly, from an imaging device such as an ultrasound probe, a CT scanner, a magnetic resonance imaging (MRI) scanner, an optical coherence tomography (OCT) scanner, an O-arm (including, for example, an O-arm 2D long film scanner), a C-arm, a G-arm, another device utilizing X-ray-based imaging (e.g., a fluoroscope or other X-ray machine), or any other imaging device. The sensor information may alternatively be or comprise information received, whether directly or indirectly, from an electromagnetic locating or navigating system, which information may comprise information about a detected position of one or more magnets or other electromagnetic field sources, from which a pose of a reference frame, a first robot, and a second robot may be determined.

**In** embodiments where the sensor information comprises image information, the image information may correspond, for example, to an x-ray image showing the reference frame, a plurality of tracking markers (e.g., tracking markers 220) of the first robot, and a plurality of tracking markers (e.g., tracking markers 220) of the second robot. The x-ray image may also show the anatomical element of the patient to which the reference frame is fixedly secured.

The sensor information may also be or comprise information from one or more sensors of the first and second robots. For example, one or more sensors of the first and second robots may be used to determine (independently of any tracking markers thereon) a pose of the robotic arms 148 thereof, which information may be utilized in the method 300. As another example, a navigation system may be used to detect any tracking markers affixed to the robotic arms of the robots and/or elsewhere on the robots, from which information a pose of the robots and/or of their robotic arms may be determined. Thus, the sensor information may be received from a navigation camera or other navigation system sensor.

Also in some embodiments, the sensor information may comprise information about a position (or, alternatively, a pose) of the second robot relative to the first robot, or vice versa (which information may be or include, for example, information from which a position or pose of the second robot relative to the first robot may be determined). For example, in embodiments where the first robot and the second robot are physically connected by a rigid member (such as, for example, the member 224), the sensor information may comprise information from a sensor in each joint (e.g., each joint 228) that connects the rigid member to one of the robots, which information may enable determination of the relative position/pose of the two robots based on known information about a position of the joints on the robots as well as about a length of the rigid member.

In some embodiments, the pose of each of the first and second robots may be a pose in which each robot is in contact with the reference frame or some other pre-determined point. Such a pose may be useful, for example, to permit determination of a precise position of one or more points of each robot based on a determined position of one or more points of the reference frame of the other predetermined point. In some embodiments, the robots may be in contact with each other at such a predetermined point. The predetermined point may be immobile relative to the base of the other robot (e.g., a robotic arm of one robot may touch the base of the other robot), or moveable relative to the base of the other robot (e.g., a robotic arm of one robot may touch the robotic arm or end effector of the other robot). One robot may, for example, "see" (using one or more sensors, such as a sensor 144) the predetermined point, and automatically move its robotic arm and/or end effector to the predetermined point. Once contact is made at the predetermined point, the position of one robot relative to the other robot is known. Also in some embodiments, the predetermined point may comprise a plurality of points that a robotic arm can contact simultaneously. Particularly where the robotic arm can contact each of the plurality of points in only one orientation, the use of a plurality of points may facilitate determination of a position of the robot. Contact between a robot and the reference frame may be utilized instead of, for example, one or more tracking markers on the robot.

The present disclosure encompasses embodiments of the method 300 that utilize variations of the step 308 (as well as the remaining steps of the method 300). For example, in some embodiments, the step 308 may comprise receiving sensor information about a pose of a reference frame and of more than two robots, and/or receiving sensor information about a pose of a reference frame, a first robot, a second robot, and the anatomical element. In each instance, the pose of the identified elements may be a simultaneous pose (e.g., a pose of each element at the same moment in time) or a sequential pose (e.g., the pose of each element at different moments in time).

The method 300 also comprises determining a correlation among a patient coordinate system corresponding to the patient, a first coordinate system corresponding to the first robot, and a second coordinate system corresponding to the second robot (step 312). The determining is based on the image information and the sensor information, although in some embodiments the determining may be based only on the sensor information. The determining the correlation may comprise registering the first and second coordinate systems (of the first and second robots, respectively) to the patient coordinate system, or registering the patient coordinate system and the first and second coordinate systems to a navigation coordinate system or other coordinate system. The determining may comprise using one or more algorithms such as the algorithms 128 to determine a mapping of any one or more of the patient coordinate system, the first coordinate system, and the second coordinate system to any others of the foregoing and/or to a navigation coordinate system or other coordinate system. The correlation may enable control of, for example, the first and second robots using instructions that reference the patient coordinate system, or may enable translation of instructions based on the patient coordinate system into instructions based on the first and/or second coordinate systems so as to cause the first and second robots to move within their respective coordinate systems to accomplish a task or procedure in the patient coordinate system. The determined correlation may be saved, for example, in a memory such as the memory 116, in a database such as the database 160, or elsewhere. The determined correlation may also be transmitted via a network such as the cloud 164.

The method 300 also comprises controlling movement of the first and second robots within a defined work volume based on the correlation (step 316). The defined work volume may comprise separate work volumes for each robot, or a single common work volume, as described elsewhere herein. Where the defined work volume is a common work volume, the common work volume may be a volume comprising points in space that are reachable and may be occupied (albeit not simultaneously) by both the first robot and the second robot. Thus, the common work volume comprises a single work volume, the entirety of which is accessible to both the first robot and the second robot, and in which the first and second robots may be safely controlled, based on the correlation, without colliding or otherwise interfering with each other. Where the defined work volume comprises separate work volumes, the separate work volumes may include a first work volume for the first robot and a second work volume for the second robot, where the first and second work volumes are mutually exclusive, thus also facilitating simultaneous operation of the first and second robots with a reduced risk of collision or interference.

The controlling may comprise causing the first and second robots (and more specifically, the robotic arms of the first and second robots, or even more specifically, the end effectors of the first and second robots) to work in a coordinated manner to accomplish a given surgical task (e.g., making an incision in a patient's skin) or a given surgical procedure (e.g., a spinal decompression). The controlling may be based, for example, on a preoperative plan, and may utilize one or more instructions such as the instructions 124 stored in a memory such as the memory 116.

The present disclosure encompasses embodiments of the method 300 that comprise more or fewer steps than those described above, and/or one or more steps that are different than the steps described above. For example, in some embodiments, the method 300 may also comprise receiving information about the first coordinate system and the second coordinate system from, for example, the first and second robots, respectively. In such embodiments, the determining step 312 may be based on the received coordinate system information, in addition to the received sensor information and/or the received image information.

Each of the coordinate systems discussed in the above discussion of the method 300 is unique from the other coordinate systems. In other words, the first robot has a first coordinate system that has a different origin than a second coordinate system of the second robot, and the first and second coordinate systems of the first and second robots, respectively, each have a different origin than the patient coordinate system. Beyond having different origins, each coordinate system, in some embodiments, may be oriented differently, such that none of the axes (e.g., none of the X, Y, or Z axes) of the first coordinate system is parallel to any of the axes of the second coordinate system and/or of the patient coordinate system (or, if any two axes are parallel, those axes are different-e.g., an X-axis of one coordinate system is parallel to a Z-axis of another coordinate system).

Turning now to Fig. 4, a method 400 of determining a correlation among a patient coordinate system (corresponding, e.g., to a patient), a first coordinate system (e.g., of a first robot), and a second coordinate system (e.g., of a second robot) may be carried out or otherwise performed, for example, by at least one processor. The at least one processor may be the same as or similar to the processor(s) 104 of the computing device 102 described above. The at least one processor may be part of a robot (such as a robot 136) or part of a navigation system (such as a navigation system 156). A processor other than any processor described herein may also be used to execute the method 400. The at least one processor may perform the method 400 by executing instructions stored in a memory, such as the instructions 124 of the memory 116. The instructions may correspond to one or more steps of the method 400 described below. The instructions may cause the processor to execute one or more algorithms, such as the algorithms 128. For example, one or more such algorithms 128 may be used to map one coordinate system to another coordinate system once each coordinate system has been located.

The method 400 corresponds to the step 312 of the method 300 described above. In other words, the method 400 constitutes one way that a correlation among a patient coordinate system, a first coordinate system of a first robot, and a second coordinate system of a second robot may be determined.

The method 400 comprises determining a first correlation between a patient coordinate system and a navigation space (step 404). The patient coordinate system may be defined relative to, for example, a point of the patient's anatomy selected by a surgeon or autonomously, and/or by a point at which a reference frame is fixedly secured to an anatomical element of the patient. The patient coordinate system may or may not be defined with respect to a surgical procedure to be performed on the patient. In some embodiments, the patient coordinate system may be defined in a preoperative plan, while in other embodiments, the patient coordinate system may be defined independently of any preoperative plan.

The navigation space may be, for example, a space visible to a sensor of a navigation system such as a navigation system 156. The navigation space, in some embodiments, may be represented by or correspond to a navigation coordinate system, and the step 404 may comprise determining a first correlation between a patient coordinate system and a navigation coordinate system.

The determining the first correlation may comprise using one or more algorithms such as the algorithms 128 to determine a mapping of the patient coordinate system to the navigation space. The first correlation may enable translation of any coordinates in the patient coordinate system to any coordinates in the navigation space, or vice versa. The first correlation may be saved, for example, in a memory such as the memory 116, in a database such as the database 160, or elsewhere. The first correlation may also be transmitted via a network such as the cloud 164.

The method 400 also comprises determining a second correlation between a first coordinate system and the navigation space (step 408). The first coordinate system is a coordinate system used by a first robot, which may be, for example, a robot 136. For example, the first robot may instruct to the robotic arm thereof to move the end effector thereof to a given set of coordinates of the first coordinate system. The first coordinate system may be defined relative to a fixed point on the robot (e.g., a point on the robot base), and/or relative to a point of an end effector of the robot when the robotic arm is in a predetermined position.

The determining the second correlation may comprise using one or more algorithms such as the algorithms 128 to determine a mapping of the first coordinate system to the navigation space. The second correlation may enable translation of any coordinates in the first coordinate system to any coordinates in the navigation space, or vice versa. The second correlation, for example, may enable control of the first robot using instructions that reference the navigation space (e.g., a navigation coordinate system), or may enable translation of instructions based on a navigation coordinate system into instructions based on the first coordinate system so as to cause the first robot to move within the first coordinate system to accomplish a task or procedure defined relative to the patient coordinate system. The second correlation may be saved, for example, in a memory such as the memory 116, in a database such as the database 160, or elsewhere. The second correlation may also be transmitted via a network such as the cloud 164.

The method 400 also comprises determining a third correlation between a second coordinate system and the navigation space (step 412). The second coordinate system is a coordinate system used by a second robot, which may be, for example, a robot 136. For example, the second robot may instruct to the robotic arm thereof to move the end effector thereof to a given set of coordinates of the second coordinate system. The second coordinate system may be defined relative to a fixed point on the robot (e.g., a point on the robot base), and/or relative to a point of an end effector of the robot when the robotic arm is in a predetermined position.

The determining the third correlation may comprise using one or more algorithms such as the algorithms 128 to determine a mapping of the second coordinate system to the navigation space. The third correlation may enable translation of any coordinates in the second coordinate system to any coordinates in the navigation space, or vice versa. The third correlation, for example, may enable control of the second robot using instructions that reference the navigation space (e.g., a navigation coordinate system), or may enable translation of instructions based on a navigation coordinate system into instructions based on the second coordinate system so as to cause the second robot to move within the second coordinate system to accomplish a task or procedure defined relative to the patient coordinate system. The third correlation may be saved, for example, in a memory such as the memory 116, in a database such as the database 160, or elsewhere. The third correlation may also be transmitted via a network such as the cloud 164.

The method 400 also comprises determining a combined correlation based on the first, second, and third correlations (step 416). The determining the combined correlation may comprise utilizing the first correlation between the patient coordinate system and the navigation space, together with the second correlation between the first coordinate system and the navigation space, to determine a mapping of the patient coordinate system to the first coordinate system, and/or vice versa. The determining may also comprise utilizing the second correlation between the first coordinate system and the navigation space and the third correlation between the second coordinate system and the navigation space to determine a mapping of the first coordinate system to the second coordinate system, and/or vice versa. The determining may further comprise utilizing the first correlation between the patient coordinate system and the navigation space, together with the third correlation between the second coordinate system and the navigation space, to determine a mapping of the patient coordinate system to the second coordinate system, and/or vice versa.

The combined correlation may enable translation of any coordinates in any one of the patient coordinate system, the first coordinate system, the second coordinate system, and/or a coordinate system corresponding to the navigation space to any other of the foregoing coordinate systems. The combined correlation may also enable control of the first and second robots using a common set of instructions prepared relative to the navigation space (e.g., a navigation coordinate system), and/or may enable translation of instructions based on a navigation coordinate system or any other coordinate system into instructions based on the first and second coordinate systems so as to cause the first and second robots, respectively, to move appropriately to accomplish a task or procedure defined relative to the patient coordinate system or the navigation coordinate system. The combined correlation may be saved, for example, in a memory such as the memory 116, in a database such as the database 160, or elsewhere. The combined correlation may also be transmitted via a network such as the cloud 164.

The present disclosure encompasses embodiments of the method 400 that comprise more or fewer steps than those described above, and/or one or more steps that are different than the steps described above.

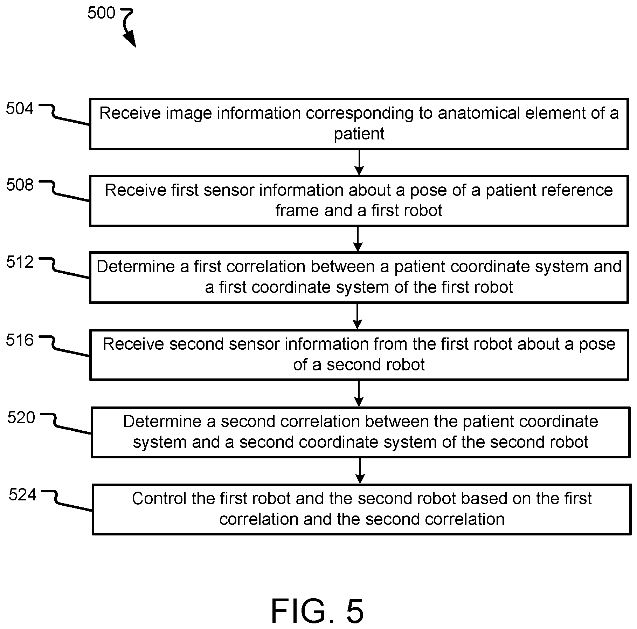

Turning now to Fig. 5, a method 500 of controlling a plurality of robots in coordinate fashion may be carried out or otherwise performed, for example, by at least one processor. The at least one processor may be the same as or similar to the processor(s) 104 of the computing device 102 described above. The at least one processor may be part of a robot (such as a robot 136) or part of a navigation system (such as a navigation system 156). A processor other than any processor described herein may also be used to execute the method 500. The at least one processor may perform the method 500 by executing instructions stored in a memory, such as the instructions 124 of the memory 116. The instructions may correspond to one or more steps of the method 500 described below. The instructions may cause the processor to execute one or more algorithms, such as the algorithms 128. For example, one or more such algorithms 128 may be used to map one coordinate system to another coordinate system once each coordinate system has been located.

The method 500 comprises receiving image information corresponding to an anatomical element of a patient (step 504). The step 504 may be the same as or similar to the step 304 of the method 300, described above.

The method 500 also comprises receiving first sensor information about a pose of a patient reference frame and a first robot (step 508). The patient reference frame may be the same as or similar to the reference frame 212, and the first robot may be the same as or similar to, for example, a robot 136 of Fig. 1, and/or a robot 136a or 136b of Figs. 2A-2B. The first sensor information may be any information useful for determining the pose of the reference frame and the first robot at a single point in time. Thus, for example, the first sensor information may comprise image information received, whether directly or indirectly, from an imaging device such as a CT scanner, a magnetic resonance imaging (MRI) scanner, an optical coherence tomography (OCT) scanner, an O-arm (including, for example, an O-arm 2D long film scanner), a C-arm, a G-arm, another device utilizing X-ray-based imaging (e.g., a fluoroscope or other X-ray machine), or any other imaging device. The first sensor information may alternatively be or comprise information received, whether directly or indirectly, from an electromagnetic locating or navigating system, which information may comprise information about a detected position of one or more magnets or other electromagnetic field sources, from which a pose of the patient reference frame and the first robot may be determined.

In embodiments where the first sensor information comprises image information, the image information may correspond, for example, to an x-ray image showing the patient reference frame and a plurality of tracking markers (e.g., tracking markers 220) of the first robot. The x-ray image may also show the anatomical element of the patient to which the patient reference frame is fixedly secured.

The first sensor information may also be or comprise information from one or more sensors of the first robot. For example, one or more sensors of the first robot may be used to determine (independently of any tracking markers thereon) a pose of a robotic arm thereof. As another example, a navigation system may be used to detect any tracking markers affixed to the robotic arm of the first robot and/or elsewhere on the first robot, from which information a pose of the robot and/or of the robotic arm may be determined. Thus, the first sensor information may be received from a navigation camera or other navigation system sensor.

The method 500 also comprises determining a first correlation between a patient coordinate system and a first coordinate system of the first robot (step 512). The patient coordinate system may be the same as or similar to any patient coordinate system described herein, including for example with respect to the step 404 of the method 400. The first coordinate system may be the same as or similar to any first coordinate system described herein, including for example with respect to the step 408 of the method 400.

The determining the first correlation may comprise using one or more algorithms such as the algorithms 128 to determine a mapping of the patient coordinate system to the first coordinate system. The first correlation may enable translation of any coordinates in the patient coordinate system to any coordinates in the first coordinate system, or vice versa. The first correlation, for example, may enable control of the first robot using instructions that reference the patient coordinate system, or may enable translation of instructions based on the patient coordinate system into instructions based on the first coordinate system so as to cause the first robot to move within the first coordinate system to accomplish a task or procedure defined relative to the patient coordinate system. The first correlation may be saved, for example, in a memory such as the memory 116, in a database such as the database 160, or elsewhere. The first correlation may also be transmitted via a network such as the cloud 164.

The method 500 also comprises receiving second sensor information from the first robot about a pose of a second robot (step 516). The second robot may be the same as or similar to, for example, a robot 136 of Fig. 1, and/or a robot 136a or 136b of Figs. 2A-2B. The second sensor information may be any information useful for determining the pose of the second robot relative to a pose of the first robot. Thus, for example, the second sensor information may comprise image information received, whether directly or indirectly, from a sensor of the first robot (e.g., a sensor 144). Such a sensor may detect, for example, one or more tracking markers (e.g., tracking markers 220) on the second robot. In embodiments where the first robot has been physically connected to the second robot via a member 224 or otherwise, the sensor may be or comprise one or more sensors configured to detect information about that physical connection, from which the pose of the second robot relative to the pose of the first robot may be determined. The second sensor information may alternatively be or comprise information received, whether directly or indirectly, from an electromagnetic locating or navigating system, which information may comprise information about a detected position of one or more magnets or other electromagnetic field sources, from which a pose of the second robot relative to a pose of the first robot may be determined.

The method 500 also comprises determining, based on the first correlation and the second sensor information, a second correlation between the patient coordinate system and a second coordinate system of the second robot (step 520). The second coordinate system may be the same as or similar to any second coordinate system described herein, including for example with respect to the step 412 of the method 400.

The determining the second correlation may comprise using one or more algorithms such as the algorithms 128 to determine (based, again, on the first correlation and the second sensor information) a mapping of the patient coordinate system to the second coordinate system. The first correlation may enable translation of any coordinates in the patient coordinate system to any coordinates in the first coordinate system, and the second sensor information may be useful for determining a pose of the second robot relative to the first robot, so as to enable determination of an intermediate correlation between the first coordinate system and the second coordinate system. The first correlation and the intermediate correlation may then be utilized to determine the second correlation. Once determined, the second correlation, for example, may enable control of the second robot using instructions that reference the patient coordinate system, or may enable translation of instructions based on the patient coordinate system into instructions based on the second coordinate system so as to cause the second robot to move within the second coordinate system to accomplish a task or procedure defined relative to the patient coordinate system. The second correlation may be saved, for example, in a memory such as the memory 116, in a database such as the database 160, or elsewhere. The second correlation may also be transmitted via a network such as the cloud 164.

The method 500 also comprises controlling movement of the first and second robots based on the first and second correlations (step 524). The controlling may comprise causing the first and second robots (and more specifically, the robotic arms of the first and second robots, or even more specifically, the end effectors of the first and second robots) to work in a coordinated manner to accomplish a given surgical task (e.g., making an incision in a patient's skin) or a given surgical procedure (e.g., a spinal decompression) defined with respect to the patient coordinate system. The controlling may be based, for example, on a preoperative plan, and may utilize one or more instructions such as the instructions 124 stored in a memory such as the memory 116.

The present disclosure encompasses embodiments of the method 500 that comprise more or fewer steps than those described above, and/or that comprise one or more steps that are different than the steps described above. For example, in some embodiments, the method 500 may also comprise determining one or more correlations between the patient coordinate system, the first coordinate system, and/or the second coordinate system, on the one hand, and a navigation coordinate system on the other hand. Also in some embodiments, the method 500 may comprise determining a correlation between each possible pair of coordinate systems, rather than just the first correlation between the patient coordinate system and the first coordinate system of the first robot and the second correlation between the patient coordinate system and the second coordinate system of the second robot.

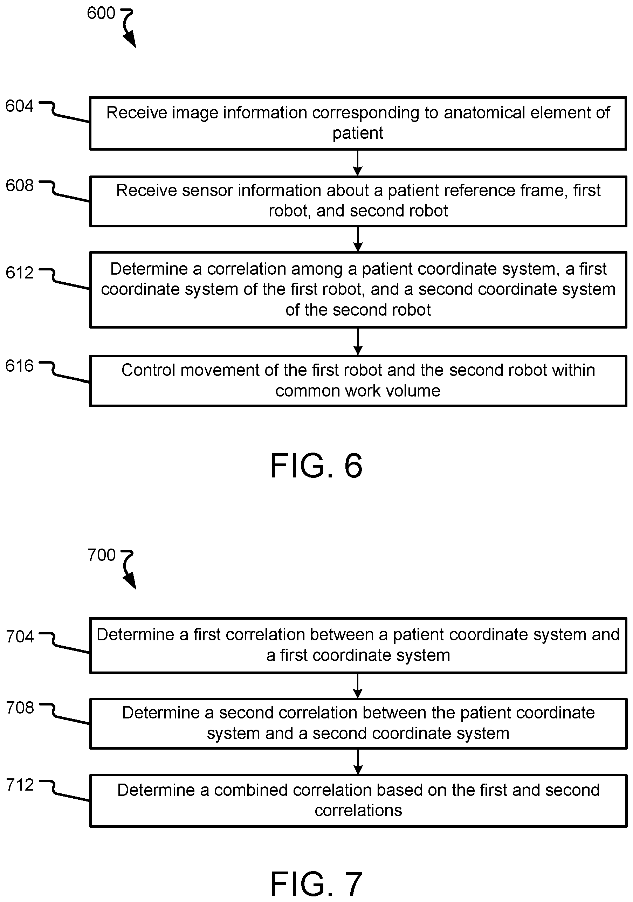

Fig. 6 depicts steps of a method 600 which may be implemented, for example, by a robot control system according to embodiments of the present disclosure. The method 600 is identical or substantially similar to the method 300 (e.g., the step 604 is identical or substantially similar to the step 304, the step 608 is identical or substantially similar to the step 308, the step 612 is identical or substantially similar to the step 312, and the step 616 is identical or substantially similar to the step 316). In particular, the received sensor information may be any sensor information sufficient (whether alone or in combination with the image information) to enable correlation among a patient coordinate system, a first coordinate system of the first robot, and a second coordinate system of the second robot, as recited in step 612. In some embodiments, the sensor information received in the step 608 may be or comprise first sensor information about the first robot and the patient reference frame (e.g., sensor information sufficient, whether alone or in combination with the image information, to enable correlation between the first coordinate system and the patient coordinate system) and second sensor information about the second robot and the patient reference frame (e.g., sensor information sufficient, whether alone or in combination with the image information, to enable correlation between the second coordinate system and the patient coordinate system).

Fig. 7 depicts steps of a method 700, which represents one way of determining a correlation among a patient coordinate system, a first coordinate system of a first robot, and a second coordinate system of a second robot during the step 612 of the method 600.

The method 700 comprises determining a first correlation between a patient coordinate system and a first coordinate system of the first robot (step 704). The step 704 may be the same as or similar to the step 512 of the method 500. The step 704 may be based, for example, on image information such as that received in the step 604 of the method 600, and sensor information such as that received in the step 608 of the method 600. Where the sensor information comprises first sensor information about a first robot and a patient reference frame, and second sensor information about a second robot and the patient reference frame, the step 704 may be based on the first sensor information.

The method 700 also comprises determining a second correlation between the patient coordinate system and a second coordinate system (step 708). The step 708 may be the same as or similar to the step 512 of the method 500, with the second coordinate system substituted for the first coordinate system. The step 708 may be based, for example, on image information such as that received in the step 604 of the method 600, and sensor information such as that received in the step 608 of the method 600. Where the sensor information comprises first sensor information about a first robot and a patient reference frame, and second sensor information about a second robot and the patient reference frame, the step 704 may be based on the second sensor information.

Alternatively, the step 708 may be the same as or similar to the step 520 of the method 500, with the determining the second correlation based on image information such as that received in the step 604 of the method 600, and/or on sensor information received from the first robot about a pose of the second robot, together with information about the first correlation determined in the step 704.

The method 700 also comprises determining a combined correlation based on the first and second correlations (step 712). The determining the combined correlation may comprise utilizing the first correlation between the patient coordinate system and the first coordinate system, together with the second correlation between the patient coordinate system and the second coordinate system, to determine a mapping of the first coordinate system to the second coordinate system, and/or vice versa. The determining may also comprise utilizing the second correlation between the first coordinate system and the navigation space and the third correlation between the second coordinate system and the navigation space to determine a mapping of the first coordinate system to the second coordinate system, and/or vice versa.

The combined correlation may enable translation of any coordinates in any one of the patient coordinate system, the first coordinate system, the second coordinate system to any other of the foregoing coordinate systems. The combined correlation may also enable control of the first and second robots using a common set of instructions prepared relative to the patient space (e.g., the patient coordinate system), and/or may enable translation of instructions based on the patient coordinate system into instructions based on the first and second coordinate systems so as to cause the first and second robots, respectively, to move appropriately to accomplish a task or procedure defined relative to the patient coordinate system. The combined correlation may be saved, for example, in a memory such as the memory 116, in a database such as the database 160, or elsewhere. The combined correlation may also be transmitted via a network such as the cloud 164.

The present disclosure encompasses embodiments of the method 700 that comprise more or fewer steps than those described above, and/or one or more steps that are different than the steps described above.

The step 612 may be completed using the method 400, the method 700, any variation on either of the preceding methods, or another method. Similarly, the step 312 may be completed using the method 400, the method 700, any variation on either of the preceding methods, or another method.

Although embodiments of the present disclosure have been described with respect to the use of two separate robots, the present disclosure also encompasses embodiments that use three, four, five, six, seven, or more separate robots. In such embodiments, the methods described herein may be readily modified to permit correlation of a coordinate system of each robot to a patient coordinate system, a navigation coordinate system, and/or other robot coordinate systems.

As may be appreciated based on the foregoing disclosure, the present disclosure encompasses methods with fewer than all of the steps identified in Figs. 3, 4, 5, 6, and 7 (and the corresponding description of the methods 300, 400, 500, 600, and 700), as well as methods that include additional steps beyond those identified in Figs. 3, 4, 5, 6, and 7 (and the corresponding description of the methods 300, 400, 500, 600, and 700). The present disclosure also encompasses methods that comprise one or more steps from one method described herein, and one or more steps from another method described herein. Any correlation described herein may be or comprise a registration or any other correlation. Any method described herein as comprising determination of one or more correlations between or among a patient coordinate system, a first coordinate system of a first robot, and a second coordinate system of a second robot may also comprise determination of one or more correlations between or among any of the foregoing and a navigation coordinate system. Similarly, any method described herein as comprising determination of one or more correlations between or among a patient coordinate system, a navigation coordinate system, a first coordinate system of a first robot, and a second coordinate system of a second robot may instead comprise determination of one or more correlations between or among only the patient coordinate system, the first coordinate system, and the second coordinate system.

The foregoing is not intended to limit the disclosure to the form or forms disclosed herein. In the foregoing Detailed Description, for example, various features of the disclosure are grouped together in one or more aspects, embodiments, and/or configurations for the purpose of streamlining the disclosure. The features of the aspects, embodiments, and/or configurations of the disclosure may be combined in alternate aspects, embodiments, and/or configurations other than those discussed above. This method of disclosure is not to be interpreted as reflecting an intention that the claims require more features than are expressly recited in each claim. Rather, as the following claims reflect, inventive aspects lie in less than all features of a single foregoing disclosed aspect, embodiment, and/or configuration. Thus, the following claims are hereby incorporated into this Detailed Description, with each claim standing on its own as a separate preferred embodiment of the disclosure.

Moreover, though the description has included description of one or more aspects, embodiments, and/or configurations and certain variations and modifications, other variations, combinations, and modifications are within the scope of the disclosure, e.g., as may be within the skill and knowledge of those in the art, after understanding the present disclosure.

## Claims

1. A robot control system (100) comprising:
a communication interface (108) for communication with a plurality of robots (136), the plurality of robots (136) comprising a first robot (136a) and a second robot (136b), with
a plurality of tracking markers (220) positioned on the first and second robots (136a, 136b), the first robot (136a) having a coordinate system independent of a coordinate system of the second robot (136b), and
at least one sensor (144) provided on the first robot (136a), the at least one sensor (144) being configured to detect a position of the second robot (136b) by detecting a position of tracking markers (220) on the second robot (136b);
at least one processor (104); and
at least one memory (116) storing instructions for execution by the at least one processor (104), the instructions, when executed, configured to cause the at least one processor (104) to:
receive sensor information regarding a patient reference frame (212), the first robot (136a), and the second robot (136b), wherein the sensor information comprises first sensor information about the first robot (136a) and the patient reference frame (212) including image information corresponding to an anatomical element of a patient, and second sensor information received from the at least one sensor provided on the first robot (136a);
determine, based on the first sensor information, a first correlation between a patient coordinate system and a first coordinate system of the first robot (136a);
determine, based on the first correlation and the second sensor information, an intermediate correlation between the first coordinate system of the first robot (136a) and a second coordinate system of the second robot (136b),
determine, based on the first correlation and the intermediate correlation, a second correlation between the patient coordinate system and the second coordinate system of the second robot (136b), and
control movement of the first robot (136a) and the second robot (136b) based on the first and second correlations.

2. The robot control system (100) of claim 1, wherein the controlling comprises causing coordinated movement of the first robot (136a) and the second robot (136b) to accomplish a surgical task.

3. The robot control system (100) of claim 1, wherein the at least one sensor (144) comprises a navigation camera.

4. The robot control system (100) of claim 1, wherein the at least one memory (116) stores additional instructions for execution by the at least one processor (104) that, when executed, further cause the at least one processor (104) to control movement of the first robot (136a) and the second robot (136b), based on the correlations, within a common work volume.

## Patentansprüche

1. Robotersteuerungssystem (100), umfassend:
eine Kommunikationsschnittstelle (108) für eine Kommunikation mit einer Vielzahl von Robotern (136), die Vielzahl von Robotern (136) umfassend einen ersten Roboter (136a) und einen zweiten Roboter (136b), mit
einer Vielzahl von Tracking-Markierungen (220), die auf dem ersten und dem zweiten Roboter (136a, 136b) positioniert sind, wobei der erste Roboter (136a) ein Koordinatensystem aufweist, das unabhängig von einem Koordinatensystem des zweiten Roboters (136b) ist, und
mindestens einen Sensor (144), der an dem ersten Roboter (136a) bereitgestellt ist, wobei der mindestens eine Sensor (144) konfiguriert ist, um eine Position des zweiten Roboters (136b) durch Erfassen einer Position von Tracking-Markierungen (220) an dem zweiten Roboter (136b) zu erfassen;
mindestens einen Prozessor (104); und
mindestens einen Speicher (116), der Anweisungen für eine Ausführung durch den mindestens einen Prozessor (104) speichert, wobei die Anweisungen, wenn ausgeführt, konfiguriert sind, um den mindestens einen Prozessor (104) zu veranlassen zum:
Empfangen von Sensorinformationen bezüglich eines Patientenreferenzrahmens (212), des ersten Roboters (136a) und des zweiten Roboters (136b),
wobei die Sensorinformationen erste Sensorinformationen über den ersten Roboter (136a) und das Patientenreferenzsystem (212), einschließlich Bildinformationen, die einem anatomischen Element eines Patienten entsprechen, und zweite Sensorinformationen umfassen, die von dem mindestens einen Sensor empfangen werden, der an dem ersten Roboter (136a) bereitgestellt ist;
Bestimmen, basierend auf den ersten Sensorinformationen, einer ersten Korrelation zwischen einem Patientenkoordinatensystem und einem ersten Koordinatensystem des ersten Roboters (136a),
Bestimmen, basierend auf der ersten Korrelation und den zweiten Sensorinformationen, einer Zwischenkorrelation zwischen dem ersten Koordinatensystem des ersten Roboters (136a) und einem zweiten Koordinatensystem des zweiten Roboters (136b),
Bestimmen, basierend auf der ersten Korrelation und der Zwischenkorrelation, einer zweiten Korrelation zwischen dem Patientenkoordinatensystem und dem zweiten Koordinatensystem des zweiten Roboters (136b), und
Steuern einer Bewegung des ersten Roboters (136a) und des zweiten Roboters (136b) basierend auf der ersten und der zweiten Korrelation.

2. Robotersteuerungssystem (100) nach Anspruch 1, wobei die Steuerung das Veranlassen einer koordinierten Bewegung des ersten Roboters (136a) und des zweiten Roboters (136b) umfasst, um eine chirurgische Aufgabe durchzuführen.

3. Robotersteuerungssystem (100) nach Anspruch 1, wobei der mindestens eine Sensor (144) eine Navigationskamera umfasst.

4. Robotersteuerungssystem (100) nach Anspruch 1, wobei der mindestens eine Speicher (116) zusätzliche Anweisungen für die Ausführung durch den mindestens einen Prozessor (104) speichert, die, wenn ausgeführt, den mindestens einen Prozessor (104) ferner veranlassen, die Bewegung des ersten Roboters (136a) und des zweiten Roboters (136b), basierend auf den Korrelationen innerhalb eines gemeinsamen Arbeitsvolumens, zu steuern.

## Revendications

1. Système de commande de robot (100) comprenant :
une interface de communication (108) permettant la communication avec une pluralité de robots (136), la pluralité de robots (136) comprenant un premier robot (136a) et un second robot (136b), avec
une pluralité de marqueurs de suivi (220) positionnés sur les premier et second robots (136a, 136b), le premier robot (136a) ayant un système de coordonnées indépendant d'un système de coordonnées du second robot (136b), et
au moins un capteur (144) installé sur le premier robot (136a), l'au moins un capteur (144) étant configuré pour détecter une position du second robot (136b) en détectant une position de marqueurs de suivi (220) sur le second robot (136b) ;
au moins un processeur (104) ; et
au moins une mémoire (116) stockant des instructions permettant l'exécution par l'au moins un processeur (104), les instructions, lorsqu'elles sont exécutées, configurées pour amener l'au moins un processeur (104) à :
recevoir des informations de capteur concernant le cadre de référence d'un patient (212), le premier robot (136a) et le second robot (136b),
dans lequel les informations de capteur comprennent des premières informations de capteur concernant le premier robot (136a) et le cadre de référence du patient (212), y compris des informations d'image correspondant à un élément anatomique d'un patient, et des secondes informations de capteur reçues à partir de l'au moins un capteur fourni sur le premier robot (136a) ;
déterminer, en fonction des premières informations de capteur, une première corrélation entre un système de coordonnées du patient et un premier système de coordonnées du premier robot (136a) ;
déterminer, en fonction de la première corrélation et des secondes informations de capteur, une corrélation intermédiaire entre le premier système de coordonnées du premier robot (136a) et le second système de coordonnées du second robot (136b),
déterminer, en fonction de la première corrélation et de la corrélation intermédiaire, une deuxième corrélation entre le système de coordonnées du patient et le second système de coordonnées du second robot (136b), et
commander le mouvement du premier robot (136a) et du second robot (136b) en fonction des première et seconde corrélations.

2. Système de commande de robot (100) selon la revendication 1, dans lequel la commande consiste à amener un mouvement coordonné du premier robot (136a) et du second robot (136b) à accomplir une tâche chirurgicale.

3. Système de commande de robot (100) selon la revendication 1, dans lequel l'au moins un capteur (144) comprend une caméra de navigation.

4. Système de commande de robot (100) selon la revendication 1, dans lequel l'au moins une mémoire (116) stocke des instructions supplémentaires permettant l'exécution par l'au moins un processeur (104) qui, lorsqu'elles sont exécutées, amènent en outre l'au moins un processeur (104) à commander le mouvement du premier robot (136a) et du second robot (136b), en fonction des corrélations, à l'intérieur d'un volume de travail commun.
